# EUROPEAN PATENT SPECIFICATION

(11) **EP 1 569 707 B1**
(45) Date of publication and mention of the grant of the patent: **25.02.2009**
(21) Application number: 03780336.8
(22) Date of filing: 04.12.2003
(51) Int. Cl.: A61M 5/168

(54) **Extravasation detector**
Vorrichtung zur Erkennung von Extravasation
Détecteur d'extravasation

(30) Priority: 10.12.2002 GB 0228770
(43) Date of publication of application: 07.09.2005
(73) Proprietor: Neorad A/S, 0256 Oslo (NO)
(72) Inventor: BRABRAND, Knut, N-1476 Rasta (NO)
(74) Representative: Jackson, Robert Patrick
(86) International application number: PCT/GB2003/005269
(87) International publication number: WO 2004/052431

(56) References cited:
- WO-A-99/15074
- US-A- 5 233 994
- US-A1- 2002 016 547
- US-A1- 2002 173 725
- US-B1- 6 425 878

## Description

The present invention relates to an apparatus and method for detecting extravasation.

There are numerous medical procedures in which it is necessary to infuse a substance into a blood vessel. Typically a cannula is inserted into a vein and the substance is fed to this via a flexible tube. The substance may be blood, saline, a drug, a contrast medium, etc. In many cases it is desirable that the infusion occurs slowly and so the substance is simply gravity fed. However, there are circumstances in which it is necessary to force the substance into the blood vessel.

One example is the infusion of a contrast medium used in conjunction with an imaging system such as angiography, computed tomography (CT), ultrasound or MRI. In many applications of these procedures it is necessary to infuse a contrast medium into the part of the body that is to be imaged. The medium must often be infused at a comparatively high rate for effective results to be achieved. As a consequence, in recent years, a number of injector-actuated syringes and power injectors for pressurized injection of contrast medium have been developed.

However, whilst such devices are valuable and effective, they do create a risk of extravasation. Extravasation is the accidental infusion of fluid such as contrast media into tissue surrounding a blood vessel, rather than into the blood vessel itself. The causes for extravasation vary. Fragile vasculature or valve disease may cause physiological limitations to the ability of the blood vessel to tolerate the high rate of fluid administration used in some procedures. In computed tomography, for example, contrast injection flow rates can be in the range of 0.1 to 10 ml/s. and so a failure of the vessel may occur. Alternatively, operator error may lead to inappropriate needle placement and patient movement may cause the infusing needle to be pulled from the intended vessel or cause the needle to be pushed through the wall of the vessel.

Extravasation of contrast media during intravenous injection is a potential serious complication that might necessitate surgical drainage of the affected region. Even though the incidence rate is low, it is considered to be a major concern and is associated with local pain and possibly necrosis of the tissue. If it' occurs during an imaging procedure it is often necessary for the examination to be aborted and repeated at a later stage. It is therefore important to be able to detect extravasation quickly and reliably so that infusion may then be stopped. Other substances may have more serious effects. Chemotherapy drugs can be toxic to tissue if not diluted by blood flow.

Several extravasation detection techniques are known in the art. Two simple and very useful techniques for detecting extravasation are palpation of the patient in the vicinity of the injection site and simple visual observation of the vicinity of the injection site by a trained health care provider.

In the palpation technique, the health care provider manually senses swelling of tissue near the injection resulting from extravasation. By visual observation, it is also sometimes possible to observe directly any swelling of the skin in the vicinity of an injection site resulting from extravasation.

There have been a number of attempts to improve the detection of extravasation. For example, mercury strain gauge plethysmographs measure the volume change resulting from venous blood flow in a cross sectional area of a limb of a patient in order to detect a change in volume of a limb or digit as a result of extravasation.

Photo-plethysmographs measure the optical scattering properties of capillary blood to detect the presence of extravasated fluids in tissue. WO 99/15074 provides a sensor pad having a surface that is placed against a patient. A light source is also provided and a detector on the pad optically detects extravasation by detecting light that is reflected, scattered, etc.

U.S. Patent No. 4,647,281 discloses subcutaneous temperature sensing of extravasation using a microwave radiometer. The temperature of the subcutaneous tissue where the fluid is injected is compared to that of the injected fluid.

It is also known to detect extravasation by measuring changes in the electrical impedance. Injection fluid in the tissue of the patient also changes the electrical impedance properties of the tissue. Thus, an impedance change of a certain level in the vicinity of the injection site is interpreted as being due to extravasation. WO 99/26686 discloses an electrode patch for attachment to the skin of a patient. It has elongate pick-up electrodes and energizing electrodes. The patch is used to monitor tissue impedance during the procedure and this is compared to a baseline level.

A disadvantage of such devices is that it can be difficult to maintain good electrical contact with the skin of the patient. Also, the location of the patch makes it more difficult to carry out palpation or visual inspection. A similar problem arises with the other prior art detectors. In order to address this problem, United States Patent No. 6,408,204 proposes an apparatus that may be positioned so as not to interfere with palpation or visual inspection. An energy source and a receiver are positioned between a first layer of high dielectric material and a second layer of low dielectric material. If extravasation occurs, as noted above, there is a change in the bulk electrical properties of the tissue. The receiver measures a signal resulting from changes in the energy supplied to the tissue by the energy source.

US 2002/0173725 discloses an adhesive Doppler pad to detect a pulse signal of a patient. Acoustic waves are applied to an artery and the waves reflected from red blood cells are received and the associated Doppler shift is calculated to thereby determine a patient's pulse.

US 6425878 discloses a method and device for detecting extravasation which employs a non-invasive sensor system on the skin to identify changes in subcutaneous interstitial fluid pressure and skin temperature.

According to the present invention there is provided a method of detecting extravasation during the intended infusion of a substance into a blood vessel characterized by the step of detecting a change in the flow velocity within the blood vessel downstream of the point of infusion.

Thus, unlike the prior art techniques the present invention is not based on volume changes of tissue induced by extravasation, but on direct monitoring of the increased flow velocity within the blood vessel induced by the infusion- The lack of a velocity increase indicates that extravasation has occurred. Since the increase in flow velocity should occur almost immediately infusion commences, this method gives the operator an early warning if a problem occurs.

It can be difficult to precisely locate the blood vessel downstream of the point of infusion. Therefore in a preferred embodiment of the invention, an array of detector elements is arranged substantially transverse to the direction of flow of the blood vessel so that at least one element of the array will be located over the vessel. The signal from each detector element varies depending on whether the detector element is located over the blood vessel or over ordinary tissue. This has the advantage that the accuracy with which the detector must be placed is reduced. At least one element of the array will be located over the vessel, and so it is not necessary to precisely locate the blood vessel at the point of measurement prior to commencing the measurement. Instead, a change in flow velocity will be detected by whichever element or elements of the array are located over the blood vessel. The change in flow velocity could be detected by measuring the flow velocity at a single point downstream of the point of infusion. In one preferred embodiment however, the flow velocity within the blood vessel is measured at a plurality of points spaced apart along the extent of the vessel and positioned downstream of the point of infusion. This has the advantage of enabling a user to determine the approximate position within the vessel at which extravasation has occurred.

Although the method of the invention is applicable to any infusion of a substance that causes a detectable increase in blood flow velocity, it is of particular use where the infusion is at a high rate where the greatest risk of extravasation occurs. The rates of infusion may be over 5ml/s and sometimes over 10ml/s. Thus, the invention is of particular application to venous infusions such as contrast agents. The invention may therefore be incorporated as part of a process of generating a medical image.

The invention could just be applied when the infusion is commenced, or when the rate of infusion is increased, these being times when a problem is most likely to occur. However, as noted above, extravasation may be caused by patient movement and so preferably blood flow velocity changes are continuously or repeatedly monitored during the procedure.

In a simple form of the invention, a change in velocity may be noted by an operator who can then stop the infusion. However, it is preferable that the method further comprises the provision of a notification that extravasation has occurred and most preferably there may be automatic shutdown of the infusion in response to the detection of extravasation.

The invention also extends to an apparatus for detecting extravasation during the intended infusion of a substance into a blood vessel comprising a detector for detecting a change in the flow velocity within the blood vessel downstream of the point of infusion, the apparatus being arranged to provide an output signal when extravasation occurs.

The output signal may be a notification such as an alarm. More preferably it comprises a control signal to control the infusion. The output signal need not be "high" to indicate extravasation. Indeed, it may be preferable to use a fail-safe system in which a "high" output indicates an increased velocity and therefore that extravasation has not occurred. Thus, if the "high" signal is lost, either due to extravasation or equipment failure, the infusion can be stopped.

Any suitable method of detecting flow velocity may be applied, but it is believed that the most effective technique is ultrasound Doppler. Thus, the detector is preferably an ultrasound Doppler probe which may consist of a single transducer element and more preferably consists of an array of individual transducer elements adapted to be arranged substantially transverse to the direction of flow of the blood vessel so that the position of the blood vessel can be detected and/or the change in flow velocity in the blood vessel can be detected without first knowing the precise location of the blood vessel. In another embodiment of the invention, a plurality of individual transducer elements (or arrays of transducer elements) are spaced apart along the direction of flow of the blood vessel to form an array (or a two dimensional array) of transducer elements which can track the direction of a blood vessel in use. The probe may be located against the skin of a patient proximate to a vein into which the infusion is being made and downstream of the infusion site. The probe is preferably fixed to the patient's skin using an adhesive. In accordance with standard practice, a coupling medium (ultrasound gel) should preferably be applied to the patient's skin under the transducer elements.

The probe may be connected to a display unit in the conventional manner in which case increases in flow velocity will be visible conventionally as bright patches on the display. These may be detected using conventional techniques, for example by comparing pixel brightness in a preselected region on the display. Alternatively, the display can be dispensed with and a direct indication of velocity produced. Normally the velocity of the infusion will be significantly higher than any other velocity of flow in the region concerned and so precise measurement is not required.

An analogue signal voltage which is generally proportional to the detected flow velocity may be provided as the output from the detector. This could be used to drive a simply calibrated meter. Additionally or alternatively the voltage may be compared to a threshold voltage such that when this is exceeded an indication is provided that extravasation has (or has not) occurred.

In many applications it may be preferable to use a digital system. If the output from the detector is not in digital form then it may be converted using a conventional analogue-digital converter. The output may then be fed to a processor such as a personal computer or a custom processor incorporated into the apparatus.

Regardless of the system used, an output control signal may then be provided to control the infusion pump. In a simple form this may operate a relay to cut the power to the pump, or if the pump is computer controlled it may be a digital control signal. Alternatively, a valve arrangement may be used to prevent flow to the vein.

It will be appreciated that the invention extends to a system for giving an infusion comprising an infusion pump arranged to infuse a substance into a blood vessel and an extravasation detector according to the apparatus defined above wherein the detector apparatus is arranged to control the infusion pump. The invention also extends to a method of giving such an infusion comprising the use of such apparatus.

Certain embodiments of the invention will now be described, by way of example only, and with reference to the accompanying drawings in which:-
Figure 1 is a schematic view of a first embodiment of the invention;
Figure 2 is a schematic view of a modified version of the Figure 1 embodiment;
Figure 3 is a schematic view of an alternative embodiment of the invention;
Figure 4 is a diagram illustrating the use of the embodiment of Figure 1 where no extravasation has occurred; and
Figure 5 is a diagram illustrating the use of the embodiment of Figure 1 where extravasation has occurred.

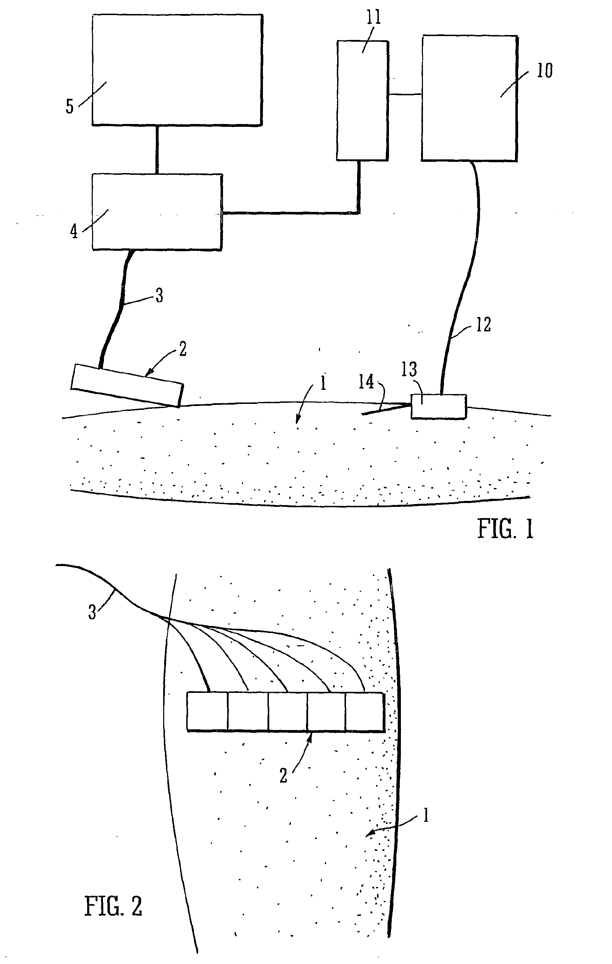

In Figure 1 a patient's arm is illustrated at 1. A contrast medium is being infused into the patient from a pump 10. The pump is controlled by an electronic pump controller 11, which varies the pump speed as required and starts and stops it.

The contrast medium flows via flexible tube 12 to cannula arrangement 13, which comprises a connector for connection to the flexible tube, and a fine bore tube 14 which has been inserted into a vein in the known manner.

Ultrasound Doppler probe 2 is placed above the same vein and a convenient distance downstream so as to be clear of the infusion site. The Doppler probe consists of a single transducer element 2 which in use is placed at an angle to the vein to create and detect a Doppler shift from the flow. The probe 2 is connected via a flexible lead 3 to a processor unit 4. This converts the output from the probe 2 into a form that may be displayed as an image on display unit 5 in the conventional manner. In addition it provides a digital signal proportional to the flow velocity detected by the probe 2. This value is then also displayed on display 5. In addition, the unit 4 determines whether the velocity corresponds to a flow of contrast medium along the vein.

When the infusion is to commence, the operator sets the desired infusion rate by inputting it into the pump controller 11 and then inputs a start signal into unit 4 by pressing a key (not shown). This in turn transmits a start signal to the pump controller 11 which energises the pump and causes it to run at the desired speed.

The processor unit 4 then checks the flow velocity as described above. If it is not satisfactory within a pre-determined short period of time the infusion will be stopped.

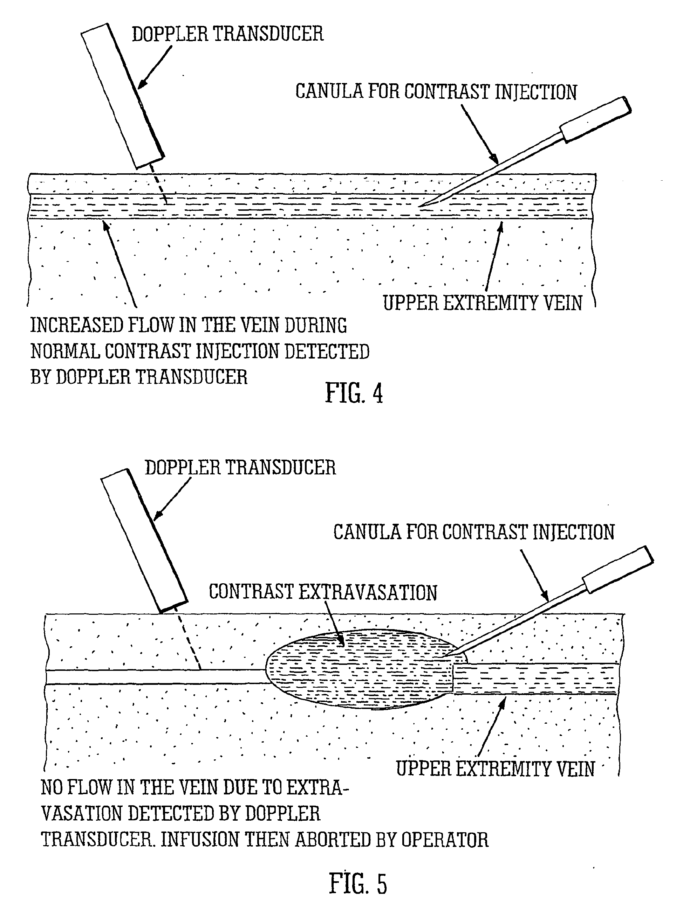

As may be seen from Figure 4, if the cannula is properly sited and the contrast medium flows as desired along the vein, this will lead to an increased flow velocity in the vein. This is detected by ultrasound probe 2 and, as described above, the processor unit 4 will therefore determine that no extravasation has occurred. It will therefore continue to send a "pump" signal to pump controller 11.

Figure 5 shows the situation that might occur when there is extravasation of contrast medium and consequently no flow in the vein. This results in a low or zero velocity output from the probe 2 from which the processor unit 4 determines that extravasation has occurred. It therefore immediately sends a "stop" signal to pump controller 11 which stops pump 10. In this way, the infusion may be stopped almost as soon as the problem occurs with the result that only a small amount of contrast medium enters the tissue surrounding the vein.

Although the situation illustrated in Fig 5 is most likely to occur when the infusion commences, the processor unit constantly monitors the output from the probe 2 throughout the infusion procedure and can stop the pump at any time.

Figure 2 shows a modified version of the embodiment of Figure 1 in which an array of individual transducer elements is provided and in use is arranged on the patient's arm substantially transverse to the direction of flow of the vein (i.e. normal to the plane of Figure 1). Thus, the precise location of the vein need not be known prior to measurement. The signal from each transducer varies depending upon whether it is situated above tissue or above a vein (a Doppler shift will be detected if the transducer is directed towards moving fluid such as blood flowing in a vein). By monitoring the signals received by each transducer element in the array, the location of the vein can be detected. Once it has been determined which transducer elements are situated over the vein, those transducer elements can be monitored for changes in the flow velocity within the vein and hence it can be determined whether or not extravasation has occurred.

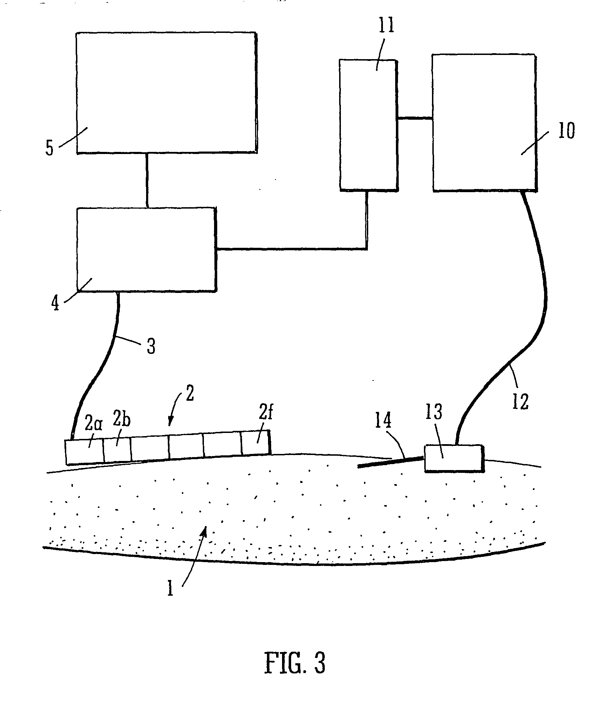

Figure 3 shows an alternative embodiment of the invention in which the Doppler probe 2 consists of a number of individual transducer elements 2a-2f. These transducer elements are spaced at regular intervals to form an array which can be placed on a patient's arm downstream of cannula arrangement 13 to extend along the vein in the flow direction.

In a modified version of the embodiment of Figure 3 (not illustrated), a two dimensional array of individual transducer elements is provided such that the elements extend both substantially transverse and substantially parallel to the direction of flow of the vein. In this way, the precise location of the vein need not be known at each measurement point before commencing measurement.

Instead, as previously described, it is determined which elements of the array are situated over the vein and those elements are monitored for changes in the flow velocity within the vein. Each such transducer element measures the flow rate at a respective point in the vein and this information is provided to the processor unit 4. The processor can therefore determine the approximate position along the vein at which extravasation has occurred. Thus for example, if the flow velocity measured at elements 2a to 2c corresponds to the flow velocity of the contrast medium within the vein but the velocity measured at element 2d does not, the processor determines that extravasation has occurred in the region of transducer element 2d. The remaining parts shown in Figure 3 correspond to those shown in Figure 1 and so are not described again here.

## Claims

1. A method of detecting extravasation during the intended infusion of a substance into a blood vessel **characterized by** the step of detecting a change in the flow velocity within the blood vessel downstream of the point of infusion.

2. A method as claimed in claim 1, wherein an array of detector elements (2) is arranged substantially transverse to the direction of flow of the blood vessel downstream of the point of infusion so as to locate at least one said detector element over the vessel.

3. A method as claimed in claim 1 or 2, wherein the flow velocity within the blood vessel is measured at a plurality of points spaced apart along the vessel and downstream of the point of infusion.

4. A method as claimed in claim 1, 2 or 3, wherein the infusion is a venous infusion of a contrast agent.

5. A method as claimed in any preceding claim, wherein flow velocity changes are continuously or repeatedly monitored during the procedure.

6. A method as claimed in any preceding claim, further comprising the provision of a notification that extravasation has occurred.

7. A method as claimed in any preceding claim, further comprising the automatic shutdown of the infusion in response to the detection of extravasation.

8. A method as claimed in any preceding claim, wherein extravasation is indicated by detecting a lack of a flow velocity increase after the intended infusion has commenced.

9. An apparatus for detecting extravasation during the intended infusion of a substance into a blood vessel comprising a detector (2), the apparatus being arranged to provide an output signal when extravasation occurs **characterized in that** the detector (2) is a detector for detecting a change in the flow velocity within the blood vessel downstream of the point of infusion.

10. An apparatus as claimed in claim 9, wherein the detector comprises an array of detector elements (2) arranged to be disposed substantially transverse to the direction of flow of the blood vessel so as to locate at least one said detector element over the vessel

11. An apparatus as claimed in claim 9 or 10, the detector being adapted to measure the flow velocity within the blood vessel at a plurality of points spaced apart along the vessel and downstream of the point of infusion.

12. An apparatus as claimed in claim 9, 10 or 11, wherein the output signal is a notification or alarm.

13. An apparatus as claimed in any of claims 9 to 12, wherein the output signal is a control signal to control the infusion.

14. An apparatus as claimed in any of claims 9 to 13, or a method as claimed in any of claims 1 to 8, wherein the detector (2) is an ultrasound Doppler probe (2).

15. An apparatus as claimed in any of claims 9 to 14, wherein the apparatus is arranged to provide an output signal when there is a lack of a velocity increase after the intended infusion has commenced.

16. An apparatus or method as claimed in claim 2 or 10, wherein the detector elements (2) are ultrasound transducer elements (2).

17. A system for giving an infusion comprising an infusion pump (10) arranged to infuse a substance into a blood vessel and an extravasation detector according to any of claims 9 to 16, wherein the detector apparatus is arranged to control the infusion pump.

## Patentansprüche

1. Verfahren zur Erkennung von Extravasation während der vorgesehenen Infusion einer Substanz in ein Blutgefäß, **gekennzeichnet durch** den Schritt der Erkennung einer Veränderung der Fließgeschwindigkeit innerhalb des Blutgefäßes stromabwärts von der Infusionsstelle.

2. Verfahren gemäß Anspruch 1, bei dem eine Anordnung von Detektorelementen (2) im Wesentlichen quer zur Fließrichtung des Blutgefäßes stromabwärts von der Infusionsstelle angeordnet ist, so dass sich mindestens ein Detektorelement über dem Gefäß befindet.

3. Verfahren gemäß Anspruch 1 oder 2, bei dem die Fließgeschwindigkeit innerhalb des Blutgefäßes an mehreren Punkten gemessen wird, die sich in Abständen voneinander entlang des Gefäßes und stromabwärts von der Infusionsstelle befinden.

4. Verfahren gemäß Anspruch 1, 2 oder 3, bei dem die Infusion eine venöse Infusion eines Kontrastmittels ist.

5. Verfahren gemäß irgendeinem der voranstehenden Ansprüche, bei dem während der Prozedur Veränderungen der Fließgeschwindigkeit kontinuierlich oder wiederholt überwacht werden.

6. Verfahren gemäß irgendeinem der voranstehenden Ansprüche, außerdem umfassend die Bereitstellung einer Benachrichtigung, dass Extravasation aufgetreten ist.

7. Verfahren gemäß irgendeinem der voranstehenden Ansprüche, außerdem umfassend den automatischen Abbruch der Infusion als Reaktion auf die Erkennung von Extravasation.

8. Verfahren gemäß irgendeinem der voranstehenden Ansprüche, bei dem Extravasation angezeigt wird durch die Erkennung des Ausbleibens einer Zunahme der Fließgeschwindigkeit, nachdem die vorgesehene Infusion begonnen hat.

9. Vorrichtung zur Erkennung' von Extravasation während der vorgesehenen Infusion einer Substanz in ein Blutgefäß umfassend einen Detektor (2), wobei die Vorrichtung dafür eingerichtet ist, ein Ausgangssignal bereitzustellen, wenn Extravasation auftritt, **gekennzeichnet dadurch, dass** der Detektor (2) ein Detektor zur Erkennung einer Veränderung der Fließgeschwindigkeit innerhalb des Blutgefäßes stromabwärts der Infusionsstelle ist.

10. Vorrichtung gemäß Anspruch 9, bei der der Detektor eine Anordnung von Detektorelementen (2) aufweist, die so angeordnet sind, dass sie sich im Wesentlichen quer zur Fließrichtung des Blutgefäßes befinden, so dass sich mindestens ein Detektorelement über dem Gefäß befindet.

11. Vorrichtung gemäß Anspruch 9 oder 10, wobei der Detektor dafür angepasst ist, die Fließgeschwindigkeit innerhalb des Blutgefäßes an mehreren Punkten zu messen, die sich in Abständen voneinander entlang des Gefäßes und stromabwärts von der Infusionsstelle befinden.

12. Vorrichtung gemäß Anspruch 9, 10 oder 11, worin das Ausgangssignal eine Benachrichtigung oder ein Alarm ist.

13. Vorrichtung gemäß irgendeinem der Ansprüche 9 bis 12, worin das Ausgangssignal ein Kontroll- bzw. Steuerungssignal zur Kontrolle bzw. Steuerung der Infusion ist.

14. Vorrichtung gemäß irgendeinem der Ansprüche 9 bis 13, oder ein Verfahren gemäß irgendeinem der Ansprüche 1 bis 8, bei dem der Detektor (2) eine Doppler-Ultraschallsonde ist.

15. Vorrichtung gemäß irgendeinem der Ansprüche 9 bis 14, worin die Vorrichtung dafür eingerichtet ist, ein Ausgangssignal bereitzustellen, wenn eine Geschwindigkeitszunahme ausbleibt, nachdem die vorgesehene Infusion begonnen hat.

16. Vorrichtung oder Verfahren gemäß Anspruch 2 oder 10, worin die Detektorelemente (2) Ultraschallwandlerelemente sind.

17. System zur Verabreichung einer Infusion umfassend eine Infusionspumpe (10), die dafür eingerichtet ist, eine Substanz in ein Blutgefäß zu infundieren, und eine Vorrichtung zur Erkennung von Extravasation gemäß irgendeinem der Ansprüche 9 bis 16, worin die Vorrichtung zur Erkennung dafür eingerichtet ist, die Infusionspumpe zu kontrollieren bzw. zu steuern.

## Revendications

1. Procédé de détection d'une extravasation au cours de la perfusion programmée d'une substance dans un vaisseau sanguin, **caractérisé par** l'étape consistant à détecter une variation de la vitesse d'écoulement à l'intérieur du vaisseau sanguin en aval du point de perfusion.

2. Procédé selon la revendication 1, dans lequel un réseau d'éléments de détection (2) est agencé sensiblement transversal à la direction de l'écoulement du vaisseau sanguin en aval du point de perfusion de manière à implanter au moins un élément de détection précité au-dessus du vaisseau.

3. Procédé selon la revendication 1 ou 2, dans lequel la vitesse d'écoulement à l'intérieur du vaisseau sanguin est mesurée au niveau d'une pluralité de points espacés le long du vaisseau et en aval du point de perfusion.

4. Procédé selon la revendication 1, 2 ou 3, dans lequel la perfusion est une perfusion veineuse d'un agent de contraste.

5. Procédé selon l'une quelconque des revendications précédentes, dans lequel les variations de la vitesse d'écoulement sont surveillées de manière continue ou à plusieurs reprises au cours de la procédure.

6. Procédé selon l'une quelconque des revendications précédentes, comprenant en outre la prévision d'une information selon laquelle une extravasation s'est produite.

7. Procédé selon l'une quelconque des revendications précédentes, comprenant en outre l'arrêt automatique de la perfusion en réponse à la détection d'une extravasation.

8. Procédé selon l'une quelconque des revendications précédentes, dans lequel une extravasation est signalée par la détection de l'absence d'une augmentation de la vitesse d'écoulement après que la perfusion programmée a débuté.

9. Appareil de détection d'une extravasation au cours de la perfusion programmée d'une substance dans un vaisseau sanguin comprenant un détecteur (2), l'appareil étant agencé de manière à fournir un signal de sortie quand une extravasation se produit, **caractérisé en ce que** le détecteur (2) est un détecteur détectant une variation de la vitesse d'écoulement à l'intérieur du vaisseau sanguin en aval du point de perfusion.

10. Appareil selon la revendication 9, dans lequel le détecteur comprend un réseau d'éléments de détection (2) agencé pour être disposé sensiblement transversal à la direction de l'écoulement du vaisseau sanguin de manière à implanter au moins un élément de détection précité au-dessus du vaisseau.

11. Appareil selon la revendication 9 ou 10, le détecteur étant apte à mesurer la vitesse d'écoulement à l'intérieur du vaisseau sanguin au niveau d'une pluralité de points espacés le long du vaisseau et en aval du point de perfusion.

12. Appareil selon la revendication 9, 10 ou 11, dans lequel le signal de sortie est une information ou une alarme.

13. Appareil selon l'une quelconque des revendications 9 à 12, dans lequel le signal de sortie est un signal de commande destiné à commander la perfusion.

14. Appareil selon l'une quelconque des revendications 9 à 13, ou procédé selon l'une quelconque des revendications 1 à 8, dans lequel le détecteur (2) est une sonde Doppler à ultrasons (2).

15. Appareil selon l'une quelconque des revendications 9 à 14, dans lequel l'appareil est agencé de manière à fournir un signal de sortie en l'absence d'une augmentation de la vitesse après que la perfusion programmée a débuté.

16. Appareil ou procédé selon la revendication 2 ou 10, dans lequel les éléments de détection (2) sont des éléments piézo-électriques à ultrasons (2).

17. Système d'administration d'une perfusion comprenant une pompe à perfusion (10), agencée de manière à perfuser une substance dans un vaisseau sanguin, et un détecteur d'extravasation selon l'une quelconque des revendications 9 à 16, dans lequel l'appareil de détection est agencé de façon à commander la pompe à perfusion.
